# EUROPÄISCHE PATENTSCHRIFT

(11) **EP 0 560 159 B1**
(45) Veröffentlichungstag und Bekanntmachung des Hinweises auf die Patenterteilung: **08.05.1996**
(21) Anmeldenummer: 93103214.8
(22) Anmeldetag: 01.03.1993
(51) Int. Cl.: C07C 68/06, C07C 69/96

(54) **Verfahren zur Herstellung von aromatischen Carbonaten**
Process for the preparation of aromatic carbonates
Procédé de préparation de carbonates aromatiques

(30) Priorität: 12.03.1992 DE 4207853
(43) Veröffentlichungstag der Anmeldung: 15.09.1993
(73) Patentinhaber: BAYER AG, D-51368 Leverkusen (DE)
(72) Erfinder: Buysch, Hans-Josef, Dr., W-4150 Krefeld (DE); Schön, Norbert, Dr., W-4150 Krefeld (DE); Rechner, Johann, Dr., W-4150 Krefeld (DE)

(56) Entgegenhaltungen:
- EP-A- 0 461 274
- DE-A- 3 308 921

## Beschreibung

Die vorliegende Erfindung betrifft ein Verfahren zur Herstellung von aromatischen Carbonaten durch Umesterung von aliphatischen Carbonaten mit phenolischen Verbindungen in Gegenwart von Titan-Verbindungen. Hierin wird das Umesterungsgemisch vor der Aufarbeitung auf eine Temperatur bevorzugt von unter 80°C abgekühlt, wobei das Gemisch noch flüssig bleiben muß, der ausgefallene, Titan enthaltende Niederschlag abgetrennt wird und das aromatische Carbonat nach an sich üblichen Methoden gewonnen wird.

Es ist bekannt, Alkylarylcarbonate und Diarylcarbonate durch Umesterung von Dialkylcarbonaten mit phenolischen Verbindungen zu gewinnen, wobei als Katalysatoren Titan-Verbindungen verwendet werden können. In DE-OS 2 528 412 und DE-OS 2 552 907 werden beispielsweise derartige katalysierte Umesterungen beschrieben. Obwohl Titan-Verbindungen an sich gut geeignete Katalysatoren sind, läßt die Selektivität, besonders bei langen Reaktionszeiten und hohen Temperaturen, noch zu wünschen übrig, wie dies beispielsweise in EP 879 bestätigt wird.

Daher ist es zweckmäßig, den Umsetzungsgrad nicht zu hoch anzusetzen, weil hierfür lange Zeiten bei hohen Temperaturen erforderlich wären; es wird vielmehr angestrebt, nach relativ niedrigen bis mittleren Umsetzungsgraden das Umesterungsgemisch aufzuarbeiten, wie dies auch in den genannten DE-Offenlegungsschriften beschrieben ist.

Zur Gewinnung der aromatischen Carbonate müssen derartige Umesterungsgemische aufgearbeitet werden. Dies kann beispielsweise durch Destillation oder Kristallisation geschehen. Kristallisationen solcher Gemische sind allerdings aufwendige Operationen, besonders, weil eine weitgehende Entfernung des Katalysators gewährleistet sein soll. Wegen dieses Erfordernisses wird man daher eine destillative Aufarbeitung vorziehen. Da hierbei hochsiedende Verbindungen voneinander getrennt werden sollen, gerät das gesamte aufzuarbeitende Reaktionsgemisch wiederum in den Bereich hoher Temperaturen mit langen Verweilzeiten, wodurch Nebenreaktionen verursacht werden, die durch die niedrigen Umesterungsgrade soeben erst vermieden werden sollten. Arbeitet man umgekehrt zur Absenkung der Destillationstemperaturen bei einem sehr guten Vakuum, muß mit großen Gasvolumina und entsprechend großen Apparaten bei hohen Investitionen gerechnet werden.

Die genannten Schwierigkeiten könnten vermieden werden, wenn man vor der Aufarbeitung den für die Nebenreaktionen verantwortlichen Katalysator aus dem Umesterungsgemisch entfernen könnte.

Überraschend wurde nun gefunden, daß die Entfernung des Titan-Katalysators auf einfache und vor allen Dingen weitgehend vollständige Weise dadurch erreicht werden kann, daß man das Reaktionsgemisch vor der Aufarbeitung auf eine Temperatur von unter 120°C, bevorzugt unter 100°C, besonders bevorzugt unter 80°C abkühlt, wobei dieses Gemisch flüssig bleiben muß. Man kann dann den ausgefallenen, Titan enthaltenden Niederschlag abtrennen. Das verbleibende Reaktionsgemisch enthält restliche Titanmengen von weniger als 100 ppm.

Es wurde ein Verfahren zur Herstellung von aromatischen Carbonaten der Formel

R¹O-CO-OR² (I)

durch Umesterung von aliphatischen Carbonaten der Formel

R³O-CO-OR⁴ (II)

mit phenolischen Verbindungen der Formel

R¹OH (III),

wobei in den Formeln
- R¹: Phenyl oder Naphthyl bedeuten, die 1- bis 3-fach durch geradkettiges oder verzweigtes C₁-C₄-Alkyl, geradkettiges oder verzweigtes C₁-C₄-Alkoxy, Fluor, Chlor, Brom oder Cyano substituiert sein können, wobei bei Mehrfachsubstitution auch verschiedene Substituenten des genannten Bedeutungsumfanges zulässig sind,
- R²: für geradkettiges oder verzweigtes C₁-C₆-Alkyl oder unabhängig von R¹ für den Bedeutungsumfang von R¹ steht,
- R³: geradkettiges oder verzweigtes C₁-C₆-Alkyl bedeutet und
- R⁴: unabhängig von R² den Bedeutungsumfang von R² annimmt,
in Gegenwart von Titan-Verbindungen der Formel

Ti(X¹, X², X³, X⁴) (IV),

in der
- X¹, X², X³ und X⁴: unabhängig voneinander Halogen, O-R⁵ oder O-CO-R⁵ bedeuten, wobei R⁵ für geradkettiges oder verzweigtes C₁-C₁₈-Alkyl oder für den unter R¹ genannten Bedeutungsumfang steht, und wobei weiter X³ und X⁴ gemeinsam 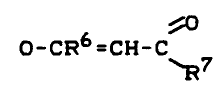 bedeuten können, worin R⁶ und R⁷ unabhängig voneinander für Methyl oder Ethyl oder OR⁵ stehen
gefunden, das dadurch gekennzeichnet ist, daß man das Umesterungsgemisch vor der Aufarbeitung auf eine Temperatur von unter 120°C, bevorzugt unter 100°C, besonders bevorzugt unter 80°C abkühlt, wobei das Gemisch noch flüssig bleiben muß, den ausgefallenen, Titan enthaltenden Niederschlag abtrennt und das aromatische Carbonat nach an sich üblichen Methoden gewinnt.

Durch die erfindungsgemäße Abkühlung des Reaktionsgemisches und die Abtrennung des ausgefallenen, Titan enthaltenden Niederschlages erhält man in einer überraschend einfachen Operation ein Reaktionsgemisch, das man sowohl durch Kristallisation als auch durch Destillation unter an sich üblichen Bedingungen zur Gewinnung des aromatischen Carbonats aufarbeiten kann, ohne Ausbeuteverlust befürchten zu müssen. Besondere Reaktionsbedingungen und besondere Vorsichtsmaßnahmen, wie oben beschrieben, sind hierbei nicht erforderlich.

Geradkettiges oder verzweigtes C₁-C₆-Alkyl ist beispielsweise Methyl, Ethyl, Propyl, Isopropyl, Butyl, Isobutyl oder Hexyl, bevorzugt Methyl oder Ethyl, besonders bevorzugt Methyl. Höheres Alkyl im Rahmen von R⁵ mit 7 bis 18 C-Atomen ist beispielsweise Heptyl, Octyl, Decyl, Dodecyl oder Octadecyl oder eines der zugehörigen verzweigten Isomeren.

Geradkettiges oder verzweigtes C₁-C₄-Alkoxy ist beispielsweise Methoxy, Ethoxy, Propoxy, Isopropoxy, Butoxy oder Isobutoxy.

Geeignete phenolische Verbindungen für das erfindungsgemäße Verfahren sind Phenol oder Naphthol beziehungsweise deren Substitutionsprodukte, wie oben angegeben. In besonderer Weise handelt es sich hierbei um Phenol, das mit C₁-C₃-Alkyl, Methoxy, Chlor oder Brom substituiert sein kann; in besonders bevorzugter Weise ist die phenolische Verbindung das nicht substituierte Phenol selbst. Beispielhaft seien die folgenden phenolischen Verbindungen genannt:
Phenol, o-, m-, p-Kresol, o-, m-, p-Ethylphenol, p-Isopropylphenol, o-, m-, p-Methoxyphenol, o-, m-, p-Chlorphenol, Bromphenol, Cyanophenol, Naphthol.

Geeignete aliphatische Carbonate für das erfindungsgemäße Verfahren enthalten mindestens eine aliphatische Gruppe; daher kann es sich hierbei sowohl um die Alkylcarbonate als auch um Alkylarylcarbonate handeln. Alkylarylcarbonate sind solche, bei denen ein Alkylrest bereits mit Hilfe einer der genannten phenolischen Verbindungen umgeestert worden ist, die aber einer Umesterung auch der zweiten Alkylgruppe fähig sind. In bevorzugter Weise werden Dialkylcarbonate eingesetzt. Solche sind beispielsweise: Dimethylcarbonat, Diethylcarbonat, Dipropylcarbonat, Diisopropylcarbonat, Dibutylcarbonat und Diisobutylcarbonat, bevorzugt Dimethyl- und Diethylcarbonat, besonders bevorzugt Dimethylcarbonat.

Erfindungsgemäß erhältliche aromatische Carbonate sind solche mit mindestens einer aromatischen Gruppe und können demzufolge Alkylarylcarbonate oder Diarylcarbonate sein. Als Alkylarylcarbonate seien beispielhaft genannt: Methyl-phenylcarbonat, Methyl-naphthylcarbonat, Ethyl-phenylcarbonat, Ethyl-naphthylcarbonat, Methyl-chlorphenylcarbonat, Methyl-methoxyphenylcarbonat und weitere, in denen Alkyl- und Arylreste gemäß obiger Beschreibung vorliegen. Diarylcarbonate sind beispielsweise Diphenylcarbonat, Dinaphthylcarbonat, Phenylnaphthylcarbonat, Bis-(chlorphenyl)-carbonat, Bis-(methoxyphenyl)-carbonat und weitere, die sich aus der obigen Beschreibung der Arylreste ergeben, besonders bevorzugt aber Methylphenylcarbonat und Diphenylcarbonat.

Es wird deutlich, daß Alkylarylcarbonate sowohl als Reaktionsprodukt gemäß Formel (I) als auch als Einsatzprodukt der Formel (II) zur Zweitumesterung fungieren können. Damit umfaßt der Begriff "Umesterung" im erfindungsgemäßen Sinn folgende Reaktionen:
a) Die Umesterung eines Dialkylcarbonats mit einer phenolischen Verbindung zu einem Alkylarylcarbonat;
b) Die Umesterung eines Alkylarylcarbonats mit phenolischer Verbindung zu einem Diarylcarbonat;
c) Die Umesterung eines Dialkylcarbonats in einer zweistufigen, im gleichen Reaktor oder in verschiedenen Reaktoren hintereinander ablaufenden Reaktion zum Diarylcarbonat über den weg der Zwischenstufe des Alkylarylcarbonats; und
d) Die Umesterung eines Alkylarylcarbonats mit einem weiteren Alkylarylcarbonats in einer Disproportionierungsreaktion zum Diarylcarbonat.

Im Falle einer zweistufigen Reaktion gemäß b) über a) können die Arylcarbonate mit zwei verschiedenen Arylresten erhalten werden. Wegen der besseren Übersichtlichkeit der Reaktion und wegen der eindeutigen Weiterverwendung der erhaltenen aromatischen Carbonate ist es jedoch bevorzugt, symmetrische aromatische Carbonate anzustreben.

Im Falle der Disproportionierungsreaktion nach d) kann diese Disproportionierung im Sinne einer weitreichenden Gewinnung des Diarylcarbonats dadurch verbessert werden, daß das entstehende Dialkylcarbonat destillativ aus dem Reaktor und damit aus dem Gleichgewicht entfernt wird. Für den Fall, daß eine Disproportionierungsreaktion nach d) im Reaktor simultan zu den Umesterungen zum Alkylarylcarbonat und zum Diarylcarbonat erfolgt, wird etwa zwischenzeitlich erhaltenes Dialkylcarbonat im erfindungsgemäßen Sinn durch vorhandene phenolische Verbindung sofort wieder in die Umesterung einbezogen.

Das erfindungsgemäße Verfahren kann selbstverständlich absatzweise bis zur Erreichung des gewünschten Umesterungsgrades durchgeführt werden, woraufhin das Umesterungsgemisch erfindungsgemäß abgekühlt wird, von dem ausgefallenen, Titan enthaltenden Niederschlag abgetrennt wird und weiter aufgearbeitet wird.

In hervorragender Weise ist das erfindungsgemäße Verfahren jedoch kontinuierlich in Umesterungskolonnen mit geeigneten Einbauten oder Füllkörpern durchführbar (vgl. EP-A-461 274). Hierbei wird der Sumpfablauf im erfindungsgemäßen Sinn behandelt, das heißt, auf eine Temperatur von unter 120°C, bevorzugt unter 100°C, besonders bevorzugt unter 80°C abgekühlt, wobei dieser Sumpfablauf noch flüssig bleiben muß, vom ausgefallenen, Titan enthaltenden Niederschlag abgetrennt und dann weiter aufgearbeitet wird.

Geeignete Titan-Verbindungen für das erfindungsgemäße Verfahren sind solche der obigen Formeln (IV), beispielsweise Titantetrachlorid, Titantetraisopropylat, Titantetrabutylat, Titantetradodecylat, Titantetraphenolat, Titantetramethoxyphenolat, Titantetrachlorphenolat, Titantetraacetat, Titantetrabutyrat, Titantetrabenzoat, Titantetraoctoat, Titan-IV-acetylacetonatdiisopropylat, Titan-IV-acetylacetonat-dibutylat, bevorzugt Titantetrachlorid, Titantetraisopropylat, Titantetrabutylat, Titantetradodecylat und Titantetraphenolat. Solche Titankatalysatoren sind dem Fachmann bekannt.

Die Einsatzmengen und die Mol-Verhältnisse der Edukte und des Katalysators werden so gewählt, wie sie dem Fachmann aus dem Stand der Technik bekannt sind, beispielsweise gemäß DE-OS 2 528 412, DE-OS 2 552 907, EP 879, EP 880 und EP-A-461 274; in analoger Weise gilt dies für die zur Umesterung anzuwendenden Temperaturen und Drücke.

Die erfindungsgemäße Abtrennung der Titan-Verbindungen aus dem Reaktionsgemisch erfolgt im allgemeinen so, daß man das Umesterungsgemisch zunächst auf eine Temperatur von unter 120°C, bevorzugt unter 100°C, besonders bevorzugt unter 80°C abkühlt. Bei niedrig schmelzenden, nicht substituierten Arylcarbonaten oder bei einem hohen Anteil von Phenol im Reaktionsgemisch kann dieses vorteilhaft auch auf unter 60°C, bevorzugt auf unter 50°C abgekühlt werden. Hierbei wird das Umesterungsgemisch flüssig gehalten, was je nach Zusammensetzung eine andere unterste Temperatur bedingt, die jedoch im Einzelfall leicht durch Vorversuche zu ermitteln ist.

Der beim Abkühlen ausfallende, Titan enthaltende Niederschlag kann nach üblichen Techniken, beispielsweise Sedimentieren, Filtrieren oder Zentrifugieren vom Umesterungsgemisch abgetrennt werden.

Der flüssige Zustand und eine geeignete Viskosität des Umesterungsgemisches können dadurch erreicht werden, daß man den Umesterungsgrad so einstellt, daß noch ausreichend Phenol und/oder aliphatisches Carbonat, bevorzugt Dialkylcarbonat, im Umesterungsgemisch anwesend ist. Flüssiger Zustand und geeignete Viskosität können aber auch durch nachträgliche Zugabe von phenolischen Verbindung und/oder aliphatischem Carbonat eingestellt werden. Schließlich ist es auch möglich, ein systemfremdes, inertes Lösungsmittel zuzusetzen. Selbstverständlich ist es auch möglich, mehrere Verbindungen aus der Gruppe phenolische Verbindung, aliphatisches Carbonat und inertes Lösungsmittel anzuwenden. Inerte, das heißt, unter den Bedingungen der Abtrennung des ausgefallenen, Titan enthaltenden Niederschlages und der folgenden Aufarbeitung nichtreaktive Lösungsmittel sind dem Fachmann bekannt und sind beispielsweise Cyclohexan, Pentan, Hexan, Octan, Isooctan, Isononan, Benzol, Toluol, Xylol, Cumol, Cymol und Mesitylen, ferner Diisopropylether, Methyl-tert.-butylether, Methyl-tert.-amylether, Dibutylether und weitere inerte Lösungsmittel.

Die Zusammensetzung des Umesterungsgemisches vor der Abtrennung des ausgefallenen, Titan enthaltenden Niederschlages kann je nach Einstellung des Umsetzungsgrades in weiten Grenzen schwanken und folgende Zusammensetzung haben:
95 bis 10 Gew.-% phenolische Verbindung,
0 bis 50 Gew.-% Dialkylcarbonat,
0 bis 80 Gew.-% Alkylarylcarbonat und
0,2 bis 90 Gew.-% Diarylcarbonat,
wobei die Summe von Alkylaryl- und Diarylcarbonat mindestens 5 Gew.-% beträgt.

In bevorzugter Weise hat das Umesterungsgemisch vor Abtrennung des ausgefallenen, Titan enthaltenden Niederschlags folgende Zusammensetzung:
90 bis 16 Gew.-% phenolische Verbindung,
0 bis 30 Gew.-% Dialkylcarbonat,
4 bis 50 Gew.-% Alkylarylcarbonat und
1 bis 60 Gew.-% Diarylcarbonat;
wobei die Summe von Alkylaryl- und Diarylcarbonat mindestens 8 Gew.-% beträgt, und besonders bevorzugt
85 bis 30 Gew.-% phenolische Verbindung,
0 bis 20 Gew.-% Dialkylcarbonat,
6 bis 40 Gew.-% Alkylarylcarbonat und
2 bis 40 Gew.-% Diarylcarbonat,
wobei die Summe von Alkylaryl- und Diarylcarbonat mindestens 10 Gew.-% beträgt.

Diese Angaben beziehen sich auf das Gesamtgewicht des aufzuarbeitenden Umesterungsgemisches ohne Hinzurechnung der Titan-Verbindung.

Für den Fall der Mitverwendung eines der oben genannten inerten Lösungsmittel beträgt dessen Menge 3 bis 50 Gew.-%, bevorzugt 5 bis 30 Gew.-%, bezogen auf die Summe der Gewichte des Umesterungsgemisches und des eingesetzten Lösungsmittels. Die Zusammensetzungen des Umesterungsgemisches zeigen die alternativen Möglichkeiten auf, die Umesterung bevorzugt zum Alkylarylcarbonat oder bevorzugt zum Diarylcarbonat zu lenken. In allen genannten Fällen ist eine effektive Abtrennung des Katalysators in erfindungsgemäßer Weise möglich, so daß der Gehalt der vom Titan-Katalysator befreiten Reaktions(Umesterung-)mischung an Titan-Katalysator weniger als 100 ppm, bevorzugt weniger als 50 ppm, besonders bevorzugt weniger als 20 ppm beträgt.

Weiterhin ist es möglich, den Titankatalysator nach Abtrennen der nicht umgesetzten Edukte und des aromatischen Carbonats durch Zugabe einer oder mehrerer phenolischer Verbindungen in Mengen von 0,5 - 20 Gew.-Teilen, bezogen auf die eingesetzte Katalysatormenge, auszufällen und dadurch zu reinigen.

Der abgetrennte, Titan enthaltende Niederschlag kann ohne weitere Reinigung in die Umesterung zurückgeführt werden. Dazu kann er als solcher oder auch nach Auflösung in den Edukten eingesetzt werden. Es ist selbstverständlich auch möglich, den abgetrennten, Titan enthaltenden Niederschlag ganz oder teilweise aus dem Verfahren zur Umesterung herauszunehmen und ihn durch frischen Katalysator zu ersetzen. Dies richtet sich in einer dem Fachmann grundsätzlich bekannten Art nach dem aktuellen Verschmutzungs- beziehungsweise Desaktivierungszustandes des Katalysators. Wegen der schonenderen Behandlung des Katalysators unter den erfindungsgemäßen Maßnahmen, wobei hohe Temperatur- und Zeitbelastungen vermieden werden, sind jedoch lange Gebrauchszeiten des Katalysators möglich.

### Beispiel 1

### Umesterung und Abtrennung eines Katalysators

Auf eine 10-bödige Glockenbodenkolonne von 60 cm Länge und 5 cm ⌀ gab man nach dem Erreichen des stationären Gleichgewichtes stündlich ein Gemisch von 250 g Phenol und 3,77 g Titantetraisopropylat am Kopf auf und dosierte am Fuß der Kolonne stündlich 250 g Dimethylcarbonat dampfförmig ein. Der Kolonnenmantel und -sumpf wurden auf 180°C, der Verdampfer für Dimethylcarbonat auf 190°C und der Dephlegmator am Kopf auf 87°C gehalten. Die Innentemperatur der Kolonne betrug 165 bis 170°C. Stündlich wurden aus dem Kolonnensumpf 250 bis 270 g Produktgemisch entnommen, das etwa folgende Zusammensetzung aufwies:
84 Gew.-% Phenol
12 Gew.-% Methylphenylcarbonat
4 Gew.-% Diphenylcarbonat
Nach 4 Stunden Laufzeit wurden die Produktfraktionen vereinigt, das vereinigte Gemisch auf 50°C abgekühlt, wobei sofort ein Niederschlag ausfiel, der durch Filtration über eine Drucknutsche abgetrennt wurde. Das klare Filtrat enthielt weniger als 5 ppm Ti; der Ausgangswert betrug 3600 ppm Ti.

Der Filterrückstand wurde entsprechend obigem Beispiel wiederverwendet, wobei das gleiche Umesterungsergebnis erhalten wurde.

### Beispiel 2

### Rückgewinnung des Katalysators aus einem Destillationssumpf

Das Beispiel 1 wurde wiederholt, wobei innerhalb von 4 Stunden aus dem Kolonnensumpf 1075 g eines Produktgemisches, bestehend aus
85 Gew.-% Phenol,
12 Gew.-% Methylphenylcarbonat,
3 Gew.-% Diphenylcarbonat
und dem Titankatalysator,
entnommen wurden. Das Produktgemisch wurde über eine 40 cm lange mit Raschigringen gefüllte Kolonne bei 25 bis 0,1 mbar destilliert. Dabei wurden das Phenol, das Methylphenylcarbonat und der größte Teil des Diphenylcarbonats über Kopf abgetrennt. Als Sumpfprodukt blieben 30,5 g einer braunen glasartigen Masse zurück (Titan-Gehalt 8,3 Gew.-%, 53 mmol). Dieses Öl wurde mit 100 g Phenol versetzt und 15 Minuten bei ca. 60°C verrührt. Dabei fielen orangefarbene Kristalle an, die isoliert, einmal mit 50 g Phenol bei 45 bis 50°C gewaschen und im Vakuum getrocknet wurden. Man erhielt 24,9 g einer Titanverbindung mit 9,2 Gew.-% Titangehalt. Das entspricht 90,3 % des eingesetzten Titans. Der so zurückgewonnene Katalysator wurde, wie im Beispiel 1 beschrieben, mit der dort verwendeten Titan-Konzentration wiederverwendet, wobei das gleiche Umesterungsergebnis wie dort erhalten wurde.

## Patentansprüche

1. Verfahren zur Herstellung von aromatischen Carbonaten der Formel
R¹O-CO-OR²
durch Umesterung von aliphatischen Carbonaten der Formel
R³O-CO-OR⁴
mit phenolischen Verbindungen der Formel
R¹OH,
wobei in den Formeln
R¹ Phenyl oder Naphthyl bedeutet, die 1- bis 3-fach durch geradkettiges oder verzweigtes C₁-C ₄-Alkyl, geradkettiges oder verzweigtes C₁-C₄-Alkoxy, Fluor, Chlor, Brom oder Cyano substituiert sein können, wobei bei Mehrfachsubstitution auch verschiedene Substituenten des genannten Bedeutungsumfanges zulässig sind,
R² für geradkettiges oder verzweigtes C₁-C₆-Alkyl oder unabhängig von R¹ für den Bedeutungsumfang von R¹ steht,
R³ geradkettiges oder verzweigtes C₁-C₆-Alkyl bedeutet und
R⁴ unabhängig von R² den Bedeutungsumfang von R² annimmt,
in Gegenwart von Titan-Verbindungen der Formel
Ti(X¹, X², X³, X⁴) ,
in der
X¹, X², X³ und X⁴ unabhängig voneinander Halogen, O-R⁵ oder O-CO-R⁵ bedeuten, wobei R⁵ für geradkettiges oder verzweigtes C₁-C₁₈-Alkyl oder für den unter R¹ genannten Bedeutungsumfang steht, und wobei weiter X³ und X⁴ gemeinsam 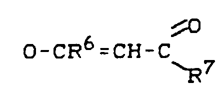 bedeuten können, worin R⁶ und R⁷ unabhängig voneinander für Methyl oder Ethyl und OR⁵ stehen,
dadurch gekennzeichnet, daß man das Umesterungsgemisch vor der Aufarbeitung auf eine Temperatur von unter 120°C, bevorzugt unter 100°C, besonders bevorzugt unter 80°C abkühlt, wobei das Gemisch noch flüssig bleiben muß, den ausgefallenen, Titan enthaltenden Niederschlag abtrennt und das aromatische Carbonat nach an sich üblichen Methoden gewinnt.

2. Verfahren nach Anspruch 1, dadurch gekennzeichnet, daß man das Umesterungsgemisch auf eine Temperatur von unter 60°C, bevorzugt von unter 50°C abkühlt.

3. Verfahren nach Anspruch 1, dadurch gekennzeichnet, daß man den flüssigen Zustand durch Anwesenheit oder durch Zugabe von phenolischer Verbindung, aliphatischem Carbonat, inerten Lösungsmittel oder einem Gemisch mehrerer von ihnen aufrecht erhält.

4. Verfahren nach Anspruch 3, dadurch gekennzeichnet, daß man folgende Zusammensetzung des Umesterungsgemisches aufrecht erhält oder einstellt:
95 bis 10 Gew.-% phenolische Verbindung,
0 bis 50 Gew.-% Dialkylcarbonat,
0 bis 80 Gew.-% Alkylarylcarbonat und
0,2 bis 90 Gew.-% Diarylcarbonat,
wobei die Summe von Alkylaryl- und Diarylcarbonat mindestens 5 Gew.-% beträgt, bevorzugt
90 bis 16 Gew.-% phenolische Verbindung,
0 bis 30 Gew.-% Dialkylcarbonat,
4 bis 50 Gew.-% Alkylarylcarbonat und
1 bis 60 Gew.-% Diarylcarbonat,
wobei die Summe von Alkylaryl- und Diarylcarbonat mindestens 8 Gew.-% beträgt, besonders bevorzugt
85 bis 30 Gew.-% phenolische Verbindung,
0 bis 20 Gew.-% Dialkylcarbonat,
6 bis 40 Gew.-% Alkylarylcarbonat und
2 bis 40 Gew.-% Diarylcarbonat,
wobei die Summe von Alkylaryl- und Diarylcarbonat mindestens 10 Gew.-% beträgt,
wobei sich die Gewichtsprozent-Angaben auf das Gesamtgewicht des aufzuarbeitenden Umesterungsgemisches ohne Anteil der Titan-Verbindung beziehen.

5. Verfahren nach Anspruch 1, dadurch gekennzeichnet, daß der abgetrennte, Titan enthaltende Niederschlag als Titan-Verbindung in die Umesterung zurückgeführt wird.

6. Verfahren nach Anspruch 1, dadurch gekennzeichnet, daß man zuerst die nicht umgesetzten Edukte und das aromatische Carbonat nach an sich üblichen Methoden abtrennt und dann den zurückbleibenden Katalysator durch Zugabe von mindestens einer phenolischen Verbindung reinigt und zurückgewinnt.

## Claims

1. Process for preparing aromatic carbonates of the formula
R¹O-CO-OR²
by transesterification of aliphatic carbonates of the formula
R³O-CO-OR⁴
with phenolic compounds of the formula
R¹OH
in which in the formulae
R¹ is phenyl or naphthyl, each of which may be mono- to tri-substituted by straight-chain or branched C₁-C₄-alkyl, straight-chain or branched C₁-C₄-alkoxy, fluorine, chlorine, bromine or cyano, different substituents from the aforementioned range of definitions also being permitted in the case of polysubstitution,
R² represents straight-chain or branched C₁-C₆-alkyl or, independently of R¹, represents the range of definitions of R¹,
R³ is straight-chain or branched C₁-C₆-alkyl, and
R⁴, independently of R², has the range of definitions of R²,
in the presence of titanium compounds of the formula
Ti(X¹, X², X³, X⁴)
in which
X¹, X², X³ and X⁴, independently of one another, halogen, are O-R⁵ or O-CO-R⁵, R⁵ representing straight-chain or branched C₁-C₁₈-alkyl or the range of definitions mentioned under R¹, and in which furthermore X³ and X⁴ together may be 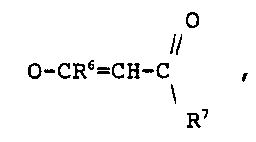 where R⁶ and R⁷, independently of one another, are methyl, ethyl or OR⁵,
characterised in that, before work-up, the transesterification mixture is cooled to a temperature of below 120°C preferably below 100°C, particularly preferably below 80°C, during which the mixture must remain liquid, the titanium-containing precipitate which is deposited is separated off, and the aromatic carbonate is obtained by methods per se which are conventional.

2. Process according to Claim 1, characterised in that the transesterification mixture is cooled to a temperature of below 60°C, preferably of below 50°C.

3. Process according to Claim 1, characterised in that the liquid state is maintained by the presence or addition of phenolic compound, aliphatic carbonate, inert solvent or a mixture of a plurality thereof.

4. Process according to Claim 3, characterised in that the following composition of the transesterification mixture is maintained or set:
95 to 10% by weight of phenolic compound,
0 to 50% by weight of dialkyl carbonate,
0 to 80% by weight of alkyl aryl carbonate, and
0.2 to 90% by weight of diaryl carbonate,
the sum of alkyl aryl carbonate and diaryl carbonate being at least 5% by weight, preferably
90 to 16% by weight of phenolic compound,
0 to 30% by weight of dialkyl carbonate,
4 to 50% by weight of alkyl aryl carbonate, and
1 to 60% by weight of diaryl carbonate,
the sum of alkyl aryl carbonate and diaryl carbonate being at least 8% by weight, particularly preferably
85 to 30% by weight of phenolic compound,
0 to 20% by weight of dialkyl carbonate,
6 to 40% by weight of alkyl aryl carbonate, and
2 to 40% by weight of diaryl carbonate,
the sum of alkyl aryl carbonate and diaryl carbonate being at least 10% by weight,
the weight percentage figures being based on the total weight of the transesterification mixture to be worked up, excluding the titanium compound.

5. Process according to Claim 1, characterised in that the separated, titanium-containing precipitate is recycled as titanium compound into the transesterification.

6. Process according to Claim 1, characterised in that first of all the unreacted starting products and the aromatic carbonate are separated off by methods per se which are conventional and then the remaining catalyst is purified and recovered by adding at least one phenolic compound.

## Revendications

1. Procédé pour la préparation de carbonates aromatiques de formule
R¹O-CO-OR²
par transestérification de carbonates aliphatiques de formule
R³O-CO-OR⁴
avec des composés phénoliques de formule
R¹OH,
dans lequel, dans les formules,
R¹ représente un groupe phényle ou un groupe naphtyle qui peuvent être substitués de 1 à 3 fois par un groupe alkyle en C₁-C₄ à chaîne droite ou ramifiée, par un groupe alcoxy en C₁-C₄ à chaîne droite ou ramifiée, par un atome de fluor, par un atome de chlore, par un atome de brome ou par un groupe cyano, dans lesquels, en cas de polysubstitution, différents substituants de la portée de signification mentionnée sont également possibles,
R² représente un groupe alkyle en C₁-C₆ à chaîne droite ou ramifiée ou possède, indépendamment de R¹, la portée de signification de R¹,
R³ désigne un groupe alkyle en C₁-C₆ à chaîne droite ou ramifiée, et
R⁴ possède, indépendamment de R², la portée de signification de R²,
en présence de composés de titane de formule
Ti(X¹, X², X³, X⁴),
dans laquelle
X¹, X², X³ et X⁴ désignent, indépendamment l'un de l'autre, un atome d'halogène, O-R⁵ ou O-CO-R⁵ où R⁵ représente un groupe alkyle en C¹-C¹⁸ à chaîne droite ou ramifiée ou encore possèdent la portée de signification mentionnée sous R¹, et dans lequel, en outre, X³ et X⁴ peuvent représenter ensemble 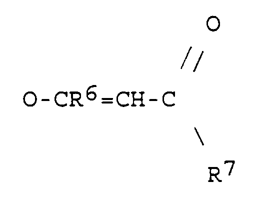 où R⁶ et R⁷ représentent, indépendamment l'un de l'autre, un groupe méthyle ou un groupe éthyle et OR⁵,
caractérisé en qu'on refroidit le mélange de transestérification avant le traitement à une température inférieure à 120°C, de préférence inférieure à 100°C, de manière particulièrement préférée inférieure à 80°C, le mélange devant encore rester liquide, on sépare le précipité obtenu contenant du titane et on obtient le carbonate aromatique conformément à des procédés habituels en soi.

2. Procédé selon la revendication 1, caractérisé en ce qu'on refroidit le mélange de transestérification à une température inférieure à 60°C, de préférence inférieure à 50°C.

3. Procédé selon la revendication 1, caractérisé en ce qu'on maintient l'état liquide par la présence ou par l'addition d'un composé phénolique, d'un carbonate aliphatique, d'un solvant inerte ou d'un mélange de plusieurs d'entre eux.

4. Procédé selon la revendication 3, caractérisé en ce qu'on maintient ou on règle la composition ci-après du mélange de transestérification :
de 95 à 10% en poids d'un composé phénolique,
de 0 à 50% en poids d'un dialkylcarbonate,
de 0 à 80% en poids d'un alkylarylcarbonate et
de 0,2 à 90% en poids d'un diarylcarbonate,
la somme de l'alkylarylcarbonate et du diarylcarbonate s'élevant à au moins 5% en poids, de préférence
de 90 à 16% en poids d'un composé phénolique,
de 0 à 30% en poids d'un dialkylcarbonate,
de 4 à 50% en poids d'un alkylarylcarbonate et
de 1 à 60% en poids d'un diarylcarbonate,
la somme de l'alkylarylcarbonate et du diarylcarbonate s'élevant à au moins 8% en poids, de manière particulièrement préférée
de 85 à 30% en poids d'un composé phénolique,
de 0 à 20% en poids d'un dialkylcarbonate,
de 6 à 40% en poids d'un alkylarylcarbonate et
de 2 à 40% en poids d'un diarylcarbonate,
la somme de l'alkylarylcarbonate et du diarylcarbonate s'élevant à au moins 10% en poids,
les indications en % en poids se rapportant au poids total du mélange de transestérification à traiter, exempt de la fraction du composé de titane.

5. Procédé selon la revendication 1, caractérisé en ce que le précipité séparé contenant du titane est recyclé dans la transestérification sous forme d'un composé de titane.

6. Procédé selon la revendication 1, caractérisé en ce que, en premier lieu, on sépare, conformément à des procédés habituels en soi, les éduits n'ayant pas réagi et le carbonate aromatique, et après , on purifie le catalyseur subsistant par addition d'au moins un composé phénolique et on le récupère.
